# EUROPEAN PATENT APPLICATION

(11) **EP 4 544 997 A1**
(43) Date of publication of application: **30.04.2025**
(21) Application number: 23206036.8
(22) Date of filing: 26.10.2023
(51) Int. Cl.: A61B 5/055, A61B 5/113, G01R 33/54

(54) **METHOD FOR ACQUIRING A MOTION DETECTION SIGNAL**

(71) Applicant: Siemens Healthineers AG, 91301 Forchheim (DE)
(72) Inventor: Gumbrecht, Rene, 96049 Bamberg (DE); Liebig, Patrick, 91052 Erlangen (DE)
(74) Representative: Siemens Healthineers Patent Attorneys

(57) **Abstract**

The invention is directed for performing a magnetic resonance scan using a motion detection system (8, 18, 6, 16, 10), wherein the method comprises transmitting Tx pilot tone signals (42) via a plurality of RF transmit antennas, and receiving at least one Rx pilot tone signal via at least one RF receive antenna (16). The Tx pilot tone signals (42) are encoded using a multiple access method, in particular a code division multiple access (CDMA) method, and the receiver (6) decodes the received at least one Rx pilot tone signal into Rx pilot tone signals originating from the different RF transmit antennas (18). The invention is also directed to an MRI system and a motion detection system.

## Description

The present invention relates to a method for acquiring a motion detection signal, a method for performing a magnetic resonance scan, a computer program, a motion detection system and a magnetic resonance imaging system.

Independent of the grammatical term usage, individuals with male, female or other gender identities are included within the term.

Patient movement or motion during a diagnostic examination or scan of medical data, in particular during radiological imaging, often causes artefacts in the acquired images. Especially Magnetic Resonance (MR) imaging is relatively slow, so that cyclical motions like respiration and cardiac movement, but also patient motion with respect to the measurement system, often occur during the scan. If the movement is known, the data acquisition can be triggered to a particular phase in the cyclical movement, or the acquired data can be corrected.

It is therefore known to acquire an ECG (electrocardiogram) of the patient during radiological imaging in order to trigger the data acquisition to a particular phase in the cardiac cycle. However, taking an ECG during an MR scan presents difficulties, in particular because of the high magnetic fields, which may cause interferences in the ECG leads. Other methods for motion detection include breathing belts, the use of two-dimensional (2D) or three-dimensional (3D) cameras, radar, and RF (radiofrequency) based methods.

The basic principle of RF based methods, also termed "pilot tone" methods, is to irradiate the body with an RF signal just outside the receive bandwidth of the MR data being acquired, e.g. outside the bandwidth of the acquired MR field of view, but within the frequency range of the MR receive system, in particular within the oversampling bandwidth which is acquired during every readout. In practice, a single RF transmitter is installed in the MR system, for example above the patient, to transmit the RF signal. This so-called pilot tone signal interacts with the human body and is received via one or multiple antennas, for example the local RF coil(s). The received pilot tone signal can be analysed to extract motion information, for example as disclosed in DE 10 2015 224 162 A1, which is herewith incorporated by reference. The advantage of RF based methods for detecting patient motion during MRI scans is that the RF transmission and reception infrastructure is already present.

However, known pilot tone methods are limited in accuracy and spatial resolution, due to the long wavelength of the pilot tone signal.

It is therefore an object of the invention to improve pilot tone methods and systems in terms of accuracy and spatial resolution.

This object is met or exceeded by a method for acquiring a motion detection signal according to claim 1, a computer program according to claim 8, a motion detection system according to claim 9 and a magnetic resonance imaging system according to claim 11.

According to a first aspect, the invention is directed to a method for acquiring a motion detection signal from a body of a subject, in particular during a radiological examination, by means of a motion detection system, wherein the motion detection system comprises a transmitter comprising a plurality of RF transmit antennas and a receiver comprising at least one RF receive antenna. The method comprises the steps of transmitting Tx pilot tone signals via the plurality of RF transmit antennas, and receiving at least one Rx pilot tone signal via the at least one RF receive antenna. The Tx pilot tone signals transmitted via the plurality of RF transmit antennas are encoded using a multiple access method, and the receiver decodes the received at least one Rx pilot tone signal into Rx pilot tone signals originating from the different RF transmit antennas, using the multiple access method.

The invention has recognised that the spatial resolution in pilot tone methods is limited by using a single RF transmit antenna. Although it is possible to use several RF transmit antennas, the spatial resolution is not improved thereby, since the receiver cannot determine from which RF transmit antenna a certain signal is coming from. To overcome this problem and allow pilot tone signals originating from different RF transmit antennas to be analysed separately, the invention proposes using a multiple access method. Multiple access methods are used in telecommunications and computer networks to allow a plurality of devices using the same transmission medium to communicate over this medium and to share its capacity. For example, in wireless networks, multiple mobile phones can use the same frequency bandwidth to communicate via the network. Multiple access methods rely on different schemes, for example code division multiple access or time division multiple access, as explained in more detail below. The invention allows to extract significantly more information from the pilot tone signal, compared to the prior art approach. If the pilot tone signals received at one or several RF receive antenna(s) can be separated into the individual signals originating from the plurality of RF transmit antennas, a plurality of unique signals can be extracted, each signal having interacted differently with the human body. This allows a significantly more accurate detection of motion. For example, when using 8 RF transmit antennas and 32 RF receive antennas, 8*32=256 unique signals can be extracted.

With this approach, different channels can be used. For example, a narrow channel or frequency band which is not interfering with the magnetic resonance data being acquired may be used.

Alternatively, the multiple access method may use a spread spectrum approach. This allows to use a wide channel or frequency band, in which the spread spectrum properties of the multiple access method are used to move the motion detection signal below the noise floor of the MR data. Thereby, the bandwidth of the pilot tone signal can overlap with bandwidth of the MR data being acquired. Spreading the signal in the frequency domain results in a signal with a wider bandwidth and thus a lower power spectral density. This allows to reduce the signal power below the noise floor while still allowing signal reconstruction by the receiver. That way, the signal is not visible in the MR data, although it uses the same signal band.

Spread spectrum techniques often make use of a sequential noise-like signal structure to spread the normally narrowband information signal over a relatively wideband radio band of frequencies. The receiver correlates the received signals to retrieve the original information signal. This approach is typically used for military applications to provide secure communication by hiding the fact that communication was even taking place.

The motion detection signal is preferably a radio-frequency (RF) signal. The RF signal may have a frequency between 20 kHz and 300 GHz, preferably between 1 MHz and 1 GHz, more preferred between about 5 MHz and 300 MHz. The motion detection signal preferably is or comprises a pilot tone signal. The Tx pilot tone signal may be continuously transmitted and the Rx pilot tone signal may be continuously acquired. The signals may be continuous wave (CW) signals. The pilot tone signal may also be acquired intermittently at sufficient time resolution to resolve the physiological movement of the subject. The motion detection signal may comprise a plurality of RF signals, as explained herein. For example, the motion detection signal may comprise a plurality of Rx pilot tone signals originating from different RF transmit antennas and received via different RF receive antenna. Each signal may be a complex RF signal, i.e. comprising both phase and amplitude. The Rx pilot tone signal is preferably acquired at the same time as the Tx pilot tone signal is being transmitted. The motion detection signal thus comprises RF signals that have interacted with a body of a subject, wherein "body" may also mean a part of a body, for example, the head, the torso or part of the torso, an organ like the heart, lungs, liver or kidney, the abdomen or a limb, such as an arm or leg. The subject may be a human or animal, in particular a patient, in particular a subject who is subjected to a radiological examination.

The method of the invention is preferably carried out during a radiological examination in order to extract movement information of the patient during the examination. The radiological examination may be performed using any medical imaging modality, for example, Computed Tomography, X-ray imaging, Positron Emission Tomography or Magnetic Resonance Imaging (MRI). The method of the invention is preferably used in MRI examinations, because the RF receive infrastructure is already present. The RF receive infrastructure comprises in particular the receiver. The receiver is adapted for receiving radiofrequency (RF) signals and comprises at least one RF antenna. It may also comprise a plurality of RF antennas, which may or may not be coils generally used for acquiring MR data. The receiver may further comprise at least one amplifier, a D/A converter, and/or further electronic components for processing the received RF signals.

The transmitter comprises a plurality of RF transmit antennas, which may or may not be MR coils used for transmitting an MR signal. Preferably, the RF transmit antennas are not the same as the at least one RF receive antenna, since the pilot tone signal is preferably acquired while it is being transmitted (unlike an MR signal). In useful embodiments, the RF transmit antennas and the at least one RF receive antenna are distributed around the body or body part of the subject, from which motion is to be detected. The antennas may for example be integrated into the examination space of the medical imaging modality, e.g. the bore of an MRI system. They may also be integrated into the patient bed and/or may be arranged in a blanket or cover or local RF coil arrangement which is placed on the body or body part.

The method comprises transmitting transmit (Tx) pilot tone signals via the plurality of RF transmit antennas, and receiving at least one receive (Rx) pilot tone signal via the at least one RF receive antenna. The Rx pilot tone signals have interacted with the body and may therefore contain motion information. The Rx pilot tone is signals transmitted via the plurality of RF transmit antennas may be continuous wave signals.

The Tx pilot tone signals are encoded using a multiple access method. The receiver is aware of the code used and is therefore able to decode the received at least one Rx pilot tone signal into Rx pilot tone signals originating from the different RF transmit antennas. In a further step, the Rx pilot tone signals may be analysed in order to extract motion information. The analysis may be performed using various methods known in connection with pilot tone methods, for example by calculating a demixing matrix by means of an Independent Component Analysis algorithm, wherein the demixing matrix calculates the independent components from the several signal components, as disclosed in EP3413076A1, incorporated herein by reference.

In order to distinguish the signals radiated from the different RF transmit antennas, each pilot tone signal is modulated or encoded in a way unique for this particular RF transmit antenna. One way to do this would be to use slightly different frequencies for each RF transmit antenna. This method, however, is not preferred, in particular when using the method during an MR examination, because the available bandwidth is limited. For example, the RF receive system of an MRI system may have a bandwidth of about 1 MHz, but most of the bandwidth is used for the magnetic resonance data, leaving only a few kHz for the pilot tone signal. This would make it difficult to broadcast even at 8 different frequencies, let alone more than that. Therefore, the multiple access method is preferably a spread spectrum method, in which a wider bandwidth is used than the individual signals would require, so that several signals may be transferred simultaneously over the same carrier frequency band, utilising different spreading codes.

Preferably, the multiple access method is a code division multiple access method (CDMA). In CDMA, each signal is modulated with a unique code word, wherein preferably the code words of the different RF transmit antennas are orthogonal to one another. The receiver knows the unique code words of all RF transmit antennas and can thus separate the signals. All RF antennas can use the same channel frequency band with this approach. For example, the Rx pilot tone signal transmitted via an RF transmit antenna is modulate with or is a certain code word consisting of ones and zeros (bits), wherein a predefined number of bits constitutes one word. Depending on the number of RF transmit antennas that need to be distinguished, the code word has to have a certain length so that each code word is distinguishable. The longer the code word, i.e., the more bits are used for each code word, the better one can separate the different pilot tone signals originating from the different RF transmit antennas from one another. In other words, the better is the suppression of the other pilot tone signals. In terms of bits, each of the (preferably orthogonal) code words may for example comprise 4 to 256 bits, preferably between 8 and 64 bits, for example 32 bits.

An advantage of code division multiple access method is that each pilot tone signal may be transmitted over a wider channel/frequency bandwidth, so that the signal-to-noise ratio is better than for example in frequency division multiple access methods. This results in a better spatial resolution of the signal.

Various variations of the CDMA may be used. For example, direct sequence CDMA may be used, which is based on direct sequence spread spectrum, as used in 3G mobile phone systems. In this method, each piece of information is represented by a sequence of several pulses. The sequence is the spreading code, and each transmit antenna uses a different spreading code. Another form of CDMA uses a frequency hopping spread spectrum technique, in which the channel frequently is changed rapidly according to a sequence that constitutes the spreading code. For example, the Bluetooth communication system uses a combination of frequency hopping and further time division multiplexing communication means.

According to an embodiment, the Tx pilot tone signals are modulated with orthogonal code words, each code word having a length of less than 50ms, preferably less than 20ms. Using code words having a length of up to 50ms, preferably between 5ms and 50ms, more preferred between 5ms and 20ms, allows to have a sufficient number of available orthogonal codes, so that each RF transmit antenna may be allocated a unique orthogonal code. The length of the code word determines the time resolution of the pilot tone signals. In order to detect physiological motion, for example, the heartbeat, it is preferable to have a time resolution of between 1ms and 50ms, preferably between 5ms and 20ms. Thereby, a heart cycle having a length of about Is can be sufficiently resolved to determine the different heart phases such as systole and diastole. If only breathing motion is to be resolved, the time resolution may even be lower, for example a time resolution and thus length of a code word of up to 50ms to 200 ms may also be acceptable.

The frequency band used for transmitting the Tx pilot tone signal may for example have a width of 2 to 20 kilohertz, preferably 4 to 16 kHz. When spreading the signal below the noise floor of the MR signal and thus not interfering with the MR signal, the full MR frequency band of typically 1MHz can be used.

According to another embodiment, the multiple access method is a time division multiple access (TDMA) method. Moreover, code division and time division multiple access techniques may be combined with one another. Preferably, the Tx pilot tone signals are transmitted via the plurality of RF transmit antennas using a time multiplexing method, wherein each RF transmit antenna is allotted at least one time slot for transmitting a Tx pilot tone signal within a block having a length of less than 50 ms, preferably less than 20 ms. Thereby, the time solution is sufficiently high to detect physiological movements such as heart motion. In other words, the TDMA method is based on a time division multiplexing scheme. TDMA provides different timeslots to the different RF transmit antennas in a cyclically repetitive pattern. For example, the 1st RF transmit antenna uses the 1st time slot, the 2nd antennas uses the 2nd time slot et cetera, until the last RF transmit antenna has transmitted its signal. An advanced form is dynamic TDMA, where an assignment of transmit antennas to timeslots varies from one cycle to the next.

According to an embodiment, the method comprises a step of extracting motion information from the received (Rx) pilot tone signals originating from the different RF transmit antennas. This is possible because the receiver is aware of the encoding scheme used by the transmitter. In particular, the receiver may include a respective electronic component which is capable of performing the encoding operation and to the modulated signal accordingly. Thereby, it is possible to extract - from each Rx pilot tone signal received from an RF receive antenna - as many Rx pilot tons signals as there are RF transmit antennas. Thus, the results may be (No. of RF receive antennas) * (no. of RF transmit antennas) signals. These signals may be combined again to increase the signal-to-noise ratio. For example, one may combine all signals received from the same transmit antenna. More preferred, the signals may be analysed so as to extract spatially selective information. For example, the movement in a specific region of interest located between an RF transmit antenna and a RF receive antenna may be captured by the pilot tone signal transmitted from that particular transmit antenna and received by the respective receive antenna.

Once the received and separated Rx pilot tone signals have been allocated to regions of interest or different body parts, the pilot tone signals may be analysed for specific motion components. For example, principal component analysis techniques may be used, as disclosed for example in EP3413076A1.

According to an embodiment, a Rx pilot tone signal originating from an RF transmit antenna located close to the RF receive antenna is disregarded when extracting motion information. This has the advantage that a signal that is not travelling through the patient, e.g. where the Tx and Rx antennas are close together, can be blanked out. These signals do not contain relevant information regarding patient motion. In this context "close" may for example mean a distance of less than 10cm, preferably less than 20cm.

According to another aspect, the invention is directed to a method for performing a magnetic resonance scan to acquire magnetic resonance data from a body of a subject by means of a magnetic resonance imaging system, wherein the magnetic resonance imaging system comprises a motion detection system which comprises a transmitter comprising a plurality of RF transmit antennas and a receiver comprising at least one RF receive antenna. The Tx pilot tone signals are RF signals just outside the bandwidth of the magnetic resonance data. The Tx pilot tone signals are transmitted and encoded according to the method of the invention. All embodiments and advantages of the method for acquiring a motion detection signal applied to the method for performing a magnetic resonance scan and vice versa.

Preferably, the method uses the RF receive infrastructure of the MRI system. The pilot tone signal uses a frequency band just outside the bandwidth of the MR are signals, which are acquired as diagnostic data. In particular, the radiological examination in this case is an MR examination, preferably an MRI examination. Therein, MR data is acquired in an MR scan and may be reconstructed to from MR image. Such a scan usually is acquired in a number of MR readouts, each using a certain MR frequency bandwidth. Tx The pilot tone signal is outside this MR frequency bandwidth, but preferably not too far away, so that the Rx pilot tone signal can still be processed by the MRI system, in particular the MR receive infrastructure, including its D/A converter, amplifiers and other electronic components.

The invention is also directed to a computer program comprising program code which, when executed by a control unit of a motion detection system, will cause the motion detection system to execute the method according to any one of the preceding claims. The motion detection system may in particular comprise a transmitter and a receiver as disclosed herein.

The invention is also directed to a digital storage medium comprising a computer program according to the invention. The digital storage medium may be any computer readable medium, for example an optical, magnetic, or solid-state storage medium. It may for example be a hard disk, a CD, a USB stick, a cloud computer, a laptop or other mobile device. All features and advantages disclosed with respect to the method also apply to the computer program and the digital storage medium and vice versa.

According to a further aspect, the invention is directed to a motion detection system comprising a transmitter comprising a plurality of RF transmit antennas and a receiver comprising at least one RF receive antenna, wherein the transmitter is configured for transmitting Tx pilot tone signals via the plurality of RF transmit antennas, and wherein the receiver is configured for receiving at least one Rx pilot tone signal via the at least one RF antenna. The transmitter is configured to encode the Tx pilot tone signals using a multiple access method, and the receiver in configured to separate the Rx pilot tone signals originating from the different RF transmit antennas. The motion detection system may be part of a medical imaging modality, such as a CT or MRI system. All features and advantages disclosed with respect to the method also apply to the motion detection system and vice versa. The motion detection system may further comprise a control unit which controls the transmitter and the receiver. In particular, the control unit may determine the multiple access method used. For example, it determines and knows the code words used by each RF transmit antenna in the code division multiple access method.

According to an embodiment, the transmitter comprises 4 to 16, preferably 6 to 10, RF transmit antennas. Thereby, a sufficiently high spatial resolution of the pilot tone signal may be achieved, while still maintaining a good signal-to-noise ratio.

According to a further aspect, the invention is directed to a magnetic resonance imaging system configured for acquiring magnetic resonance data from a body of a patient, the magnetic resonance imaging system comprising a motion detection system according to the invention. All features and advantages disclosed with respect to the method and the motion detection system also apply to the magnetic resonance imaging system and vice versa.

According to an embodiment, the RF transmit antennas are dedicated pilot tone transmit antennas which are fixedly arranged within a bore of the magnetic resonance imaging system. It is advantageous to have exclusive RF transmit antennas for the Tx pilot tone signal, because it allows the MR coils to be used for the detection of the Rx pilot tone signals. Thereby, the accuracy of the received pilot tone signals is increased. The RF transmit antennas can be arranged at locations where they are close to those parts of the body where patient movement is most likely to occur, in particular close to the abdomen, specifically close to the heart and lungs.

The invention shall now be described by means of embodiments with reference to the attached drawings. In the drawings,
- Fig. 1: shows a schematic cross section through an MRI system including a motion detection system according to an embodiment of the invention;
- Fig. 2: is a flow diagram illustrating a method according to an embodiment of the invention,
- Fig. 3: illustrates four Tx pilot tone signals encoded according to a Code Division Multiple Access method.

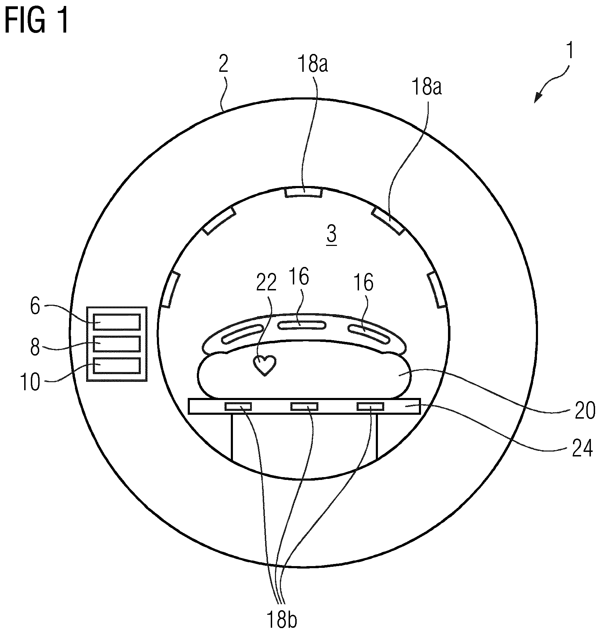

Fig. 1 is a schematic cross-sectional illustration of an MRI system 1 having a motion detection system according to an embodiment of the invention. The MRI system 1 includes a main magnet 2, which houses the (usually superconducting) coils which generate the main magnetic field, as well as the gradient coils and optionally an RF body coil for transmitting and/or receiving MR signals. The main magnet 2 encloses a bore 3 and therewith a sensitive area, in which MR scans can be performed. A patient 20 is illustrated lying on a patient bed 24, wherein the patient's heart 22 is moving in a cyclical motion while the MR examination is being carried out. The MR data of the MR scan is acquired using local RF coils 16. These RF coils 16 form a coil array which is arranged in a blanket which may be positioned on the patient's body 20.

The motion detection system includes 8 RF transmit antennas 18 arranged around the patient body 20. Three RF transmit antennas 18b are integrated into the patient bed 24. Five further RF transmit antennas 18a are arranged above the patient 20 and are attached to a housing of the main magnet 2. In the example shown, they are attached on the side facing the patient 20. However, the RF transmit coils 18a may also be covered by the housing, so that they are not visible to the patient 20. The RF transmit antennas 18 are part of a transmitter, which also includes a transmit control unit 6. The transmit control unit 6 is here shown separately from the RF transmit antennas, but within the housing of the main magnet 2. However, the transmit control unit of the transmitter may also be arranged close to the RF transmit antennas 18, for example in the patient bed 24. The transmit control unit 6 may include a signal generator, an amplifier and a D/A-converter in order to generate the RF pilot tone signal which is to be transmitted via RF transmit antennas 18. The pilot tone signal is thus encoded using a multiple access method by the transmit control unit 6, and radiated into the bore 3 via the RF transmit antennas 18. The signal is being received by RF receive antenna 16, which in this case are also receiving the MR signal. The thus received Rx pilot tone signal is transferred to the receive control unit 8, where it is amplified and may be further processed, for example decoded to thereby separate the Rx pilot tone signals originating from the different RF transmit antennas 18. The motion detection process and optionally the MR scan is controlled by a control unit 10. The control unit 10 can further comprise a digital storage medium storing the computer program which controls the multiple access method.

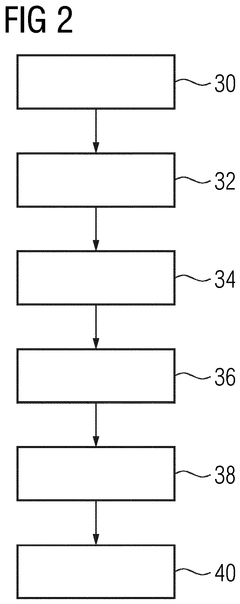

Fig. 2 illustrates the step of a method according to an embodiment of the invention. In step 30, the Tx pilot tone signals for the plurality of RF transmit antennas 18 is encoded using a predetermined multiple access scheme. In step 32, the encoded Tx pilot tone signals are transmitted via the plurality of RF transmit antennas. In step 34, the respective RF signal, which may have interacted with the patient body 20, is received by the RF receive antenna or antennas 16. In step 36, the Rx pilot tone signal or signals are decoded to thereby separate the Rx pilot tone signals originating from the different RF transmit antennas 18. In step 38, further processing of the Rx pilot tone signals in order to extract a motion detection signal is carried out, for example filtering and principal component analysis. In step 40, the respective motion detection signal is used to trigger a radiological/MR scan and/or correct data acquired during the radiological examination.

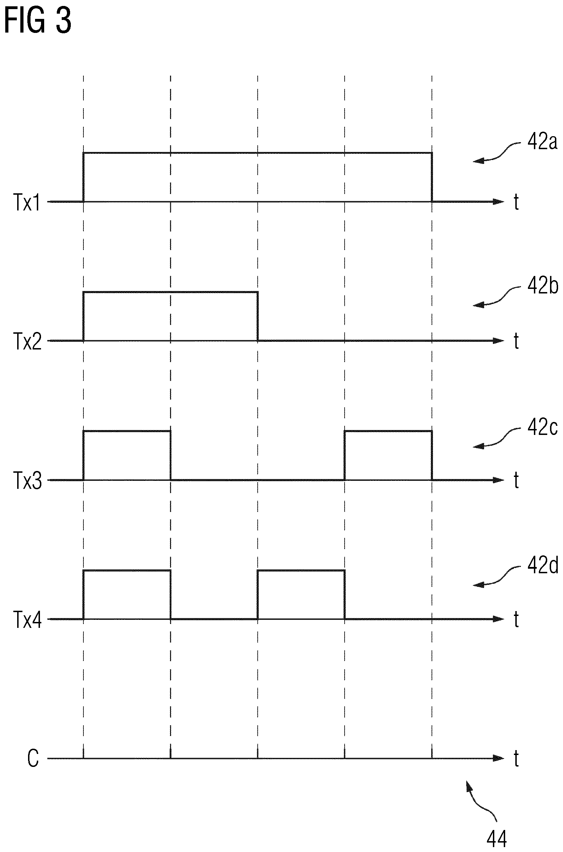

Fig. 3 illustrates the encoding of the Tx pilot tone signals. The graph shows four signals, Tx1, Tx2, Tx3, Tx4, which are transmitted by four RF transmit antennas 18. The graph shows the modulation with orthogonal code words, wherein the underlying carrier frequency is a frequency adjacent to the receive bandwidth of the MR scan. The illustrated code words each have 4 bits, wherein a so-called Walsh-code is used. The method is a synchronous CDMA method, in which all participants use the same clock. Thus, both transmitter 8 and receiver 6 are clocked by the signal 44 illustrated at the bottom of Fig. 3.

## Claims

1. Method for acquiring a motion detection signal from a body of a subject, in particular during a radiological examination, by means of a motion detection system, wherein the motion detection system comprises a transmitter (8, 18) comprising a plurality of RF transmit antennas (8) and a receiver (6, 16) comprising at least one RF receive antenna (6),
wherein the method comprises transmitting Tx pilot tone signals (42) via the plurality of RF transmit antennas (18), and receiving at least one Rx pilot tone signal via the at least one RF receive antenna (16),
**characterised in that** the Tx pilot tone signals (42) transmitted via the plurality of RF transmit antennas (18) are encoded using a multiple access method, and **in that** the receiver decodes the received at least one Rx pilot tone signal into Rx pilot tone signals originating from the different RF transmit antennas (18), using the multiple access method.

2. The method of claim 1, wherein the multiple access method is a spread-spectrum method, in particular a code division multiple access method.

3. The method of claim 2, wherein the Tx pilot tone signals (42) are modulated with orthogonal code words (42a, 42b, 42c, 42d), each code word having a length of less than 50ms, preferably less than 20ms.

4. The method of claim 1, wherein the multiple access method is a time division multiple access method.

5. The method of claim 4, wherein the Tx pilot tone signals are transmitted via the plurality of RF transmit antennas using a time multiplexing method, wherein each RF transmit antenna is allotted at least one time slot for transmitting a Tx pilot tone signal within a block having a length of less than 50ms, preferably less than 20ms.

6. The method of one of the preceding claims, comprising a step of extracting motion information from the Rx pilot tone signals originating from the different RF transmit antennas (18) .

7. The method of claim 6, wherein a Rx pilot tone signal originating from an RF transmit antenna (18) located close to a RF receive antenna (16) is disregarded when extracting motion information.

8. Method for performing a magnetic resonance scan to acquire magnetic resonance data from a body of a subject by means of a magnetic resonance imaging system (1), wherein the magnetic resonance imaging system comprises a motion detection system (8, 18, 6, 16, 10) which comprises a transmitter comprising a plurality of RF transmit antennas (18) and a receiver comprising at least one RF receive antenna (16),
wherein the method comprises transmitting Tx pilot tone signals (42) via the plurality of RF transmit antennas, the Tx pilot tone signals (42) being RF signals outside of the bandwidth of the magnetic resonance data or below the MR signal noise floor, and receiving at least one Rx pilot tone signal via the at least one RF receive antenna (16),
**characterised in that** the Tx pilot tone signals (42) transmitted via the plurality of RF transmit antennas (18) are encoded using a multiple access method, and **in that** the receiver (6) decodes the received at least one Rx pilot tone signal into Rx pilot tone signals originating from the different RF transmit antennas (18), using the multiple access method.

9. A computer program comprising program code which, when executed by a control unit (10) of a motion detection system, will cause the motion detection system to execute the method according to any one of the preceding claims.

10. A motion detection system (8, 18, 6, 16, 10) comprising a transmitter (8, 18) comprising a plurality of RF transmit antennas (18) and a receiver (6, 16) comprising at least one RF receive antenna (16),
wherein the transmitter is configured for transmitting Tx pilot tone signals (42) via the plurality of RF transmit antennas (18),
and wherein the receiver (6, 16) is configured for receiving at least one Rx pilot tone signal via the at least one RF antenna,
**characterised in that** the transmitter (8, 10) is configured to encode the Tx pilot tone signals (42) using a multiple access method, and **in that** the receiver in configured to separate the Rx pilot tone signals originating from the different RF transmit antennas.

11. A motion detection system of claim 10, wherein the transmitter comprises 4 to 16, preferably 6 to 10, RF transmit antennas (18).

12. A magnetic resonance system (1) configured for acquiring magnetic resonance data from a body of a patient (20), the magnetic resonance imaging system comprising a motion detection system (8, 18, 6, 16, 10) according to any one of claims 10 or 11,
wherein the Tx pilot tone signals (42) are RF signals outside of the bandwidth of the magnetic resonance data or below the MR signal noise floor.

13. Magnetic resonance system (1) of claim 12, wherein the RF transmit antennas (18) are dedicated pilot tone transmit antennas which are fixedly arranged within a bore (3) of the magnetic resonance system.
